# NOUVEAU FASCICULE DE BREVET EUROPEEN

(11) **EP 0 876 410 B2**
(45) Date de publication et mention de la décision concernant l'opposition: **17.08.2011**
(45) Mention de la délivrance du brevet: 03.05.2000
(21) Numéro de dépôt: 97901036.0
(22) Date de dépôt: 10.01.1997
(51) Int. Cl.: C08F 14/06, C07C 409/34

(54) **Procédé de polymérisation en suspension aqueuse du chlorure de vinyle à l'aide de peroxydicarbonates de dialkyle en solution et procédé de fabrication d'une solution de peroxydicarbonate de dialkyle**
Verfahren zur wässrigen Suspensionspolymerisation von Vinyl Chlorid mittels Dialkylperoxidicarbonate in Lösung und Verfahren zur Herstellung einer Lösung aus Dialkylperoxydicarbonat
Process for aqueous suspension polymerisation of vinyl chloride using dialkylperoxydicarbonates in solution and preparation process of a solution of dialkylperoxydicarbonate

(30) Priorité: 25.01.1996 BE 9600070
(43) Date de publication de la demande: 11.11.1998
(73) Titulaire: SOLVAY (Société Anonyme), 1050 Bruxelles (BE)
(72) Inventeur: BODART, Vincent, B-5001 Namur (BE)
(74) Mandataire: Jacques, Philippe
(86) Numéro de dépôt international: PCT/EP1997/000164
(87) Numéro de publication internationale: WO 1997/027229

(56) Documents cités:
- DD-A1- 118 608
- FR-A- 2 352 839
- FR-A- 2 466 480
- GB-A- 1 055 985
- GB-A- 1 107 956
- GB-A- 2 022 104
- GB-A- 2 024 224
- JP-A- 58 087 101
- LU-A- 44 879
- US-A- 3 377 373
- US-A- 3 799 916
- US-A- 3 935 243
- US-A- 3 950 375
- GÄCHTER R.: 'Plastics Additives Handbook 4th Edition', 1993, HANSER /GARDNER, CINCINNATI pages 382-385 - 403

## Description

La présente invention concerne un procédé de polymérisation en suspension aqueuse du chlorure de vinyle à l'aide de peroxydicarbonates de dialkyle. Elle concerne plus particulièrement un tel procédé dans lequel des peroxydicarbonates de dialkyle à courtes chaînes alkyles sont mis en oeuvre sous la forme d'une solution. L'invention concerne également un procédé de fabrication d'une solution de peroxydicarbonate de dialkyle à courtes chaînes alkyles.

Il est connu de recourir à des peroxydicarbonates de dialkyle pour initier la polymérisation en suspension aqueuse du chlorure de vinyle. Les peroxydicarbonates de dialkyle à courtes chaînes alkyles, tels que les peroxydicarbonates de diéthyle et de diisopropyle, constituent des initiateurs particulièrement appréciés du fait de leur activité élevée aux températures usuelles de polymérisation du chlorure de vinyle. Ils présentent toutefois l'inconvénient d'être instables, de sorte que leur stockage à l'état pur présente des risques très sérieux.

En vue de pallier cet inconvénient, on a déjà proposé de fabriquer ces peroxydicarbonates dans le réacteur de polymérisation ("in situ"), par exemple, par mise en réaction d'haloformiate d'alkyle dissous dans du chlorure de vinyle avec un composé peroxy, tel que le peroxyde d'hydrogène, dissous dans de l'eau alcaline. Ce procédé de fabrication "in situ" de l'initiateur ne permet pas une automatisation de l'alimentation en initiateur des réacteurs de polymérisation. En outre, il manque de reproductibilité (manque de précision sur les quantités d'initiateur effectivement mises en oeuvre à la polymérisation) et de productivité (nécessité de faire précéder chaque cycle de polymérisation par la synthèse "in situ" de l'initiateur).

On a également déjà proposé de préparer la quantité juste nécessaire de peroxydicarbonate de dialkyle en dehors du réacteur de polymérisation ("ex situ") et immédiatement avant la polymérisation.

Cette préparation s'effectue par réaction d'un haloformiate d'alkyle avec un composé peroxy en présence d'eau et d'un solvant volatil non miscible à l'eau ayant, de préférence, une température d'ébullition inférieure à 100 °C, tel que le pentane ou l'hexane. La solution d'initiateur ainsi obtenue est alors introduite in toto (phase organique et phase aqueuse) dans le réacteur de polymérisation lequel est ensuite chargé en vue de la polymérisation (brevet britannique 1 484 675 au nom de SOLVAY & Cie). Ce procédé permet l'automatisation de l'alimentation en initiateur des réacteurs mais nécessite toujours de produire la quantité juste suffisante d'initiateur immédiatement avant la polymérisation. En outre, il ne permet pas (non plus) une introduction en différé des peroxydicarbonates de dialkyle, technique intéressante par exemple pour améliorer la cinétique de la polymérisation. De plus, tout comme le procédé précité de fabrication "in situ", il produit des polymères du chlorure de vinyle conduisant, après transformation, à des articles finis contenant de nombreux yeux-de-poisson ("fish- eyes").

Les demandes de brevet britannique 2 022 104 et français 2 352 839 mentionnent des procédés de polymérisation en suspension aqueuse du chlorure de vinyle à l'aide de peroxydicarbonates de dialkyle à courtes chaînes alkyles en présence respectivement d'un plastifiant ou d'un ester d'alcool supérieur de diacide.

Le brevet américain 3 950 375 divulgue un procédé continu de préparation de peroxydicarbonates de dialkyle à l'état pur par centrifugation de la phase aqueuse de réaction.

Le brevet américain 3 377 373 décrit un procédé continu de préparation d'une solution de peroxydicarbonate de diisopropyle dans le tétrachlorure de carbone.

La présente invention a pour but de procurer un procédé de polymérisation en suspension aqueuse du chlorure de vinyle à l'aide de peroxydicarbonates de dialkyle à courtes chaînes alkyles qui ne présente aucun des inconvénients précités. Elle vise également à procurer un procédé perfectionné pour la fabrication d'une solution de peroxydicarbonates de dialkyle particulièrement adaptée à la mise en oeuvre de la polymérisation en suspension aqueuse du chlorure de vinyle.

A cet effet, l'invention concerne un procédé de polymérisation en suspension aqueuse du chlorure de vinyle à l'aide de peroxydicarbonates de dialkyle à courtes chaînes alkyles, caractérisé en ce que le peroxydicarbonate de dialkyle est mis en oeuvre sous la forme d'une solution dans un alkanedicarboxylate de dialkyle liquide et insoluble dans l'eau.

La solution de peroxydicarbonate de dialkyle à courtes chaînes alkyles mise en oeuvre selon le procédé de la présente invention est constituée essentiellement de peroxydicarbonate de dialkyle et de solvant (alkanedicarboxylate de dialkyle). Elle est donc exempte d'autres ingrédients de la polymérisation, tels que par exemple de monomère.

Par alkanedicarboxylate de dialkyle (ci-après désigné brièvement par le vocable "ester") liquide et insoluble dans l'eau, on entend désigner les esters liquides et insolubles dans l'eau dans les conditions normales, c'est-à-dire à température ambiante et sous pression atmosphérique. Par insoluble dans l'eau, on entend plus particulièrement une solubilité dans l'eau à température ambiante inférieure à 0,5 g/l. De préférence, la solubilité dans l'eau des esters servant de solvant pour le peroxydicarbonate dans le procédé de l'invention ne dépasse pas 0,3 g/l.

Les esters liquides et insolubles dans l'eau mis en oeuvre dans le procédé de l'invention présentent en général des températures d'ébullition (dans les conditions normales) largement supérieures à 100 °C. Le plus souvent elles sont supérieures à 150 °C.

A titre d'exemples d'esters utilisables, on peut mentionner les esters liquides et insolubles dans l'eau tels que définis ci-dessus dérivés d'acides alkanedicarboxyliques en C₄ à C₁₀ et d'alkanols (alcools aliphatiques saturés linéaires ou ramifiés) en C₂ à C₁₂. Parmi ceux-ci, on peut citer par exemple les butanedicarboxylates (succinates) de diéthyle et de dibutyle, les hexanedicarboxylates (adipates) de diéthyle, de dipropyle, de dibutyle, de diisobutyle et de diéthylhexyle, les octanedicarboxylates (subérates) de diéthyle et de dibutyle et les décanedicarboxylates (sébacates) de dibutyle, de diéthylbutyle et de diéthylhexyle.

Des esters convenant bien pour l'exécution du procédé de l'invention sont les alkanedicarboxylates dérivés d'acides alkanedicarboxyliques en C₄ à C₈ et d'alkanols en C₆ à C₁₀. Des esters tout particulièrement préférés sont choisis parmi les hexanedicarboxylates (adipates) dérivés d'acide adipique et d'alkanols en C₆ à C₁₀. Un ester tout particulièrement préféré dans le procédé de l'invention est l'adipate de diéthylhexyle.

La concentration en peroxydicarbonate de dialkyle des solutions mises en oeuvre dans le procédé de polymérisation selon l'invention s'élève généralement à environ 15 à 40 % en poids. La mise en oeuvre de solutions diluées de peroxydicarbonate, par exemple de solutions contenant environ 10 % en poids (ou moins) de peroxydicarbonate de dialkyle présente le risque de conduire à des polymères du chlorure de vinyle dont la température de transition vitreuse et donc la thermorésistance soit réduite. Généralement, on ne dépasse pas environ 40 % en poids, car une concentration trop élevée réduit la précision de la mesure lors de l'alimentation du réacteur en initiateur. De bons résultats sont obtenus avec des solutions dont la concentration en peroxydicarbonate de dialkyle s'élève à environ 25 à 35 % en poids.

Les solutions de peroxydicarbonates de dialkyle à courtes chaînes alkyles mises en oeuvre dans le procédé de polymérisation selon l'invention peuvent être stockées sans risque à basse température (en-dessous de 10 °C) et ce, pendant de nombreuses heures, sans perte d'activité notable. Elles peuvent dès lors être préparées à l'avance en quantité suffisante pour alimenter plusieurs réacteurs de polymérisation ou encore pour alimenter plusieurs cycles de polymérisation dans un même réacteur.

Par peroxydicarbonates de dialkyle à courtes chaînes alkyles, on entend désigner aux fins de la présente invention, les peroxydicarbonates dont les radicaux alkyles contiennent 2 ou 3 atomes de carbone et représentent les radicaux éthyle, propyle ou isopropyle, plus particulièrement les radicaux éthyle et isopropyle. Un peroxydicarbonate tout particulièrement préféré est le peroxydicarbonate de diéthyle.

Suivant un mode de réalisation particulièrement préféré du procédé de l'invention, on met dès lors en oeuvre un peroxydicarbonate de diéthyle ou de diisopropyle sous la forme d'une solution dans un hexanedicarboxylate (adipate) dérivé d'acide adipique et d'un alkanol en C₆ à C₁₀.

Il est entendu qu'outre les peroxydicarbonates de dialkyle à courtes chaînes alkyles,d'autres initiateurs usuels peuvent être mis en oeuvre conjointement dans le procédé de polymérisation de l'invention. A titre d'exemples de pareils autres initiateurs, on peut mentionner les peroxydes de dilauroyle et de dibenzoyle, les composés azoïques ou encore les peroxydicarbonates de dialkyle à longues chaînes alkyles, tels que le peroxydicarbonate de dicétyle. On préfère néanmoins initier la polymérisation exclusivement à l'aide de peroxydicarbonates de dialkyle à courtes chaînes alkyles. Contrairement aux autres peroxydes prémentionnés, ceux-ci présentent l'avantage que leurs résidus ou excès éventuellement présents dans le milieu de polymérisation à la fin du cycle de polymérisation (et qui pourraient affecter la stabilité thermique des polymères du chlorure de vinyle issus du procédé) sont aisément détruits par simple alcalinisation du milieu en fin de cycle de polymérisation.

Il est également entendu que les peroxydicarbonates de dialkyle en solution organique peuvent être introduits, en tout ou en partie après le début de la polymérisation (en différé). La mise en oeuvre en différé d'une partie du peroxydicarbonate de dialkyle à courte chaînes alkyles est avantageuse pour améliorer la cinétique de la polymérisation ou encore pour produire des résines à bas nombre K (produites à température élevée) présentant une bonne stabilité thermique. La quantité totale d'initiateur mise en oeuvre va généralement d'environ 0,15 à 0,90, et plus particulièrement encore d'environ 0,20 à 0,35 %o en poids environ par rapport au(x) monomère(s) mis en oeuvre.

Mise à part la particularité de la mise en oeuvre d'un peroxydicarbonate de dialkyle à courtes chaînes alkyles (C₂ ou C₃) sous la forme d'une solution dans un ester, les conditions générales de la polymérisation sont celles usuellement mises en oeuvre pour la polymérisation en discontinu du chlorure de vinyle en suspension aqueuse.

Par polymérisation du chlorure de vinyle, on entend désigner aux fins de la présente invention aussi bien l'homopolymérisation du chlorure de vinyle, que sa copolymérisation avec d'autres monomères éthyléniquement insaturés polymérisables par voie radicalaire. A titre d'exemples de comonomères usuels du chlorure de vinyle pouvant être mis en oeuvre dans le procédé de l'invention, on peut citer les oléfines, les oléfines halogénées, les éthers vinyliques, les esters vinyliques tels que par exemple l'acétate de vinyle, ainsi que les esters, nitriles et amides acryliques. Les comonomères sont mis en oeuvre de préférence en des quantités n'excédant pas 50 % molaires, le plus souvent 35 % molaires du mélange de comonomères mis en oeuvre. Le procédé selon l'invention convient bien pour l'homopolymérisation du chlorure de vinyle.

Par polymérisation en suspension aqueuse, on entend la polymérisation à l'aide d'initiateurs oléosolubles, en l'occurence notamment des peroxydicarbonates de dialkyle à courtes chaînes alkyles, en présence d'agents dispersants, tels que par exemple, des éthers cellulosiques hydrosolubles, des polyacétates de vinyle partiellement saponifiés (encore appelés alcools polyvinyliques) et leurs mélanges. On peut aussi, en même temps que les agents dispersants mettre en oeuvre des agents tensioactifs. La quantité d'agent dispersant mise en oeuvre varie généralement entre 0,7 et 2,0 ‰ en poids par rapport au(x) monomère(s).

La température de polymérisation est habituellement comprise entre environ 40 et 80 °C.

En fin de polymérisation, les polymères du chlorure de vinyle produits selon le procédé de l'invention sont isolés de manière conventionnelle de leur milieu de polymérisation, généralement après avoir été soumis à une épuration en monomère(s) résiduaire(s).

Le procédé de polymérisation de l'invention permet une automatisation de l'alimentation des réacteurs. Il conduit à une amélioration de la reproductibilité des cycles de polymérisation. Par ailleurs, la mise en oeuvre des peroxydicarbonates de dialkyle sous la forme d'une solution dans un ester selon l'invention n'affecte pas significativement la cinétique de la polymérisation, ni les propriétés générales (telles que nombre K, masse volumique et granulométrie) des polymères du chlorure de vinyle produits. En outre, ceux-ci procurent par transformation en fondu des articles façonnés présentant un nombre très réduit d'yeux-de-poisson.

La présente invention concerne également un procédé perfectionné de fabrication en deux étapes d'une solution de peroxydicarbonate de dialkyle à courtes chaînes alkyles utilisable dans (et particulièrement adaptée à ) la polymérisation en suspension aqueuse du chlorure de vinyle.

Suivant ce procédé, on fabrique, dans une première étape, un peroxydicarbonate de dialkyle à courtes chaînes alkyles ( tel que défini plus haut) par mise en réaction dans l'eau de quantités appropriées d'halogénoformiate d'alkyle avec un peroxyde inorganique en présence d'un sel inorganique en quantité suffisante pour augmenter la densité du milieu aqueux de réaction et, dans une deuxième étape, on sépare le peroxydicarbonate de dialkyle fabriqué par extraction au moyen d'un solvant insoluble dans l'eau, pour produire une solution de peroxydicarbonate de dialkyle dans ce solvant; le procédé étant caractérisé en outre en ce qu'on ajoute le solvant d'extraction au milieu de réaction aqueux après terminaison de la réaction de fabrication du peroxydicarbonate de dialkyle,on laisse décanter les phases et on sépare la phase organique surnageante de la phase aqueuse de réaction pour recueillir une solution pure de peroxydicarbonate.

Avantageusement, on met en oeuvre le sel inorganique en quantité suffisante pour amener la densité du milieu aqueux de réaction à une valeur au moins égale à 1,05 et plus particulièrement encore à une valeur au moins égale à 1,10. Par ailleurs, il convient d'adapter la quantité de sel inorganique de telle sorte qu'elle ne dépasse pas la concentration de saturation en sel du milieu aqueux de réaction.

La nature du sel mis en oeuvre au stade de la fabrication du peroxydicarbonate de dialkyle n'est pas particulièrement critique. En principe, tout sel inorganique qui n'interfère pas avec la réaction de formation du peroxydicarbonate de dialkyle et qui ne précipite pas dans les conditions de réaction convient. A titre d'exemples non limitatifs de tels sels, on peut citer par exemple les halogénures et en particulier les chlorures des métaux alcalins et alcalinoterreux. De préférence, on utilise des chlorures des métaux alcalins. Suivant un mode de réalisation particulièrement avantageux, on utilise du chlorure de sodium.

Le fait de réaliser la fabrication du peroxydicarbonate dans un milieu aqueux densifié conduit in fine à améliorer l'efficacité de la séparation du peroxydicarbonate de dialkyle en solution.

La particularité essentielle de la première étape est la mise en oeuvre d'un sel inorganique en quantité suffisante pour augmenter la densité de la phase aqueuse de réaction.

La température de réaction s'élève le plus souvent à une valeur située entre -10°C et + 10°C. En général, la fabrication du peroxydicarbonate est complète après quelques minutes de réaction; généralement la durée de réaction n'excède pas 10 minutes et le plus souvent 5 minutes.

L'halogénoformiate d'alkyle est le plus souvent et avantageusement un chloroformiate. Le peroxyde inorganique est le plus souvent du peroxyde de calcium ou de sodium ou encore de l'eau oxygénée. Dans ce dernier cas, il convient d'introduire en outre dans le milieu aqueux de réaction une base, telle que l'hydroxyde de calcium ou encore l'hydroxyde de sodium.

Il est particulièrement avantageux de mettre en réaction du chloroformiate d'alkyle avec du peroxyde de sodium ou encore du peroxyde d'hydrogène en présence d'hydroxyde de sodium comme base ( ce qui conduit à la formation de chlorure de sodium comme sous-produit) et de recourir, par ailleurs, à du chlorure de sodium comme sel inorganique pour densifier la phase aqueuse. Dans ce cas, la phase aqueuse saline récupérée ultérieurement (après séparation par extraction d'une solution de peroxydicarbonate de dialkyle) peut sans inconvénient être recyclée ( événtuellement après dilution) à la fabrication d'une nouvelle quantité de solution de peroxydicarbonate de dialkyle.

Cette manière de procéder présente le double avantage de réduire substantiellement la consommation en sel inorganique pour densifier la phase aqueuse et de réduire, voire éliminer, les problèmes environnementaux liés à l'évacuation de la phase aqueuse saline après la fabrication du peroxydicarbonate de dialkyle.

La nature du solvant insoluble dans l'eau utilisé dans la deuxième étape pour l'extraction du peroxydicarbonate de dialkyle n'est pas particulièrement critique. Par solvant insoluble dans l'eau, on entend désigner un solvant insoluble dans l'eau à température ambiante et sous pression atmosphérique et, plus particulièrement un solvant dont la solubilité dans l'eau dans ces conditions est inférieure à 0,5 g/l et plus particulièrement encore à 0,3g/l.

A titre d'exemples non limitatifs de solvants utilisables pour l'extraction du peroxydicarbonate de dialkyle, on peut mentionner les composés organiques insolubles dans l'eau choisis parmi les plastifiants usuels du polychlorure de vinyle. A titre d'exemples non limitatifs de tels solvants, on peut mentionner les esters d'acides polycarboxyliques aromatiques (comme les phtalates de dibutyle ou de diéthylhexyle), les époxycarboxylates d'alkyle (comme l'époxystéarate d'octyle), les huiles époxydées (comme l'huile de soja époxydée) ou encore les alkanedicarboxylates de dialkyle dont la définition est fournie plus haut dans le cadre de la description des solutions de peroxydicarbonate de dialkyle mises en oeuvre à la polymérisation en suspension aqueuse du chlorure de vinyle.

Il est particulièrement avantageux de choisir un solvant qui présente par ailleurs une densité inférieure à 1, et de préférence inférieure à 0,95.

Des solvants particulièrement préférés sont choisis parmi les alkanedicarboxylates de dialkyle dérivés d'acides alkanedicarboxyliques en C₄ à C₈ et d'alkanols en C₆ à C₁₀. Des solvants tout particulièrement préférés sont choisis parmi les hexanedicarboxylates (adipates) dérivés d'acide adipique et d'alkanols en C₆ à C₁₀. D'excellents résultats sont obtenus avec l'adipate de diéthylhexyle (température d'ébullition sous pression atmosphérique : 214 °C, solubilité dans l'eau à température ambiante : <0,2 g/l, densité : 0,922).

La quantité de solvant utilisée pour l'extraction n'est pas critique. Il va de soi qu'elle dépendra notamment du degré de solubilité du peroxydicarbonate de dialkyle dans le solvant choisi. Avantageusement, cette quantité sera telle que la concentration finale de la solution de peroxydicarbonate de dialkyle s'élève à environ 15 à environ 40% en poids et plus particulièrement encore à 25 à 35% en poids.

La deuxième étape de fabrication des solutions de peroxydicarbonates de dialkyle, à savoir la séparation par extraction du peroxydicarbonate de dialkyle fabriqué à la première étape, s'effectue de toute manière connue et appropriée.

Il est impératif de n'ajouter le solvant d'extraction au milieu de réaction aqueux qu'après la fin de la réaction de formation du peroxydicarbonate. Il a en effet été constaté que lorsque le solvant est présent dès le début de la réaction, sa présence a pour effet de ralentir la réaction et d'influer sur la pureté des solutions de peroxydicarbonate produites in fine. En pratique, on ne procédera donc à l'addition du solvant qu'au plus tôt environ 5 minutes après le début de la réaction.

Suivant un mode de réalisation tout particulièrement préféré et avantageux, on fabrique une solution de peroxydicarbonate de dialkyle à courtes chaînes alkyles (tel que de diéthyle, de dipropyle ou de diisopropyle) contenant de 15 à 40% en poids de peroxydicarbonate de dialkyle par mise en oeuvre, à la première étape du procédé de fabrication, de chlorure de sodium comme sel inorganique pour augmenter la densité de la phase aqueuse et, à la deuxième étape, d'adipates d'alkanols en C₆ à C₁₀, en particulier d'adipate de diéthylhexyle, comme solvant d'extraction pour produire une solution de peroxydicarbonate de dialkyle.

L'invention concerne également un procédé de fabrication d'une solution de peroxydicarbonate de dialkyle dont les radicaux alkyles contiennent 2 ou 3 atomes de carbone selon lequel, dans une première étape, on fabrique un peroxydicarbonate de dialkyle dont les radicaux alkyles contiennent 2 ou 3 atomes de carbone par mise en réaction dans l'eau de quantités appropriées d'halogénioformiate d'alkyle avec un peroxyde inorganique en présence d'un sel inorganique en quantité suffisante pour augmenter la densité du milieu aqueux de réaction et, dans une deuxième étape, on sépare le peroxydicarbonate de dialkyle fabriqué par extraction au moyen d'un solvant insoluble dans l'eau, choisi parmi les composés organiques insolubles dans l'eau choisis parmi les plastifiants usuels du polychlorure de vinyle, pour produire une solution de peroxydicarbonate de dialkyle dans ce solvant.

Le procédé de fabrication de solutions de peroxydicarbonates de dialkyle selon l'invention procure des solutions pures et stables au stockage avec des rendements élevés. Ces solutions peuvent être véhiculées sans danger et ne conduisent pas à des problèmes de dépôts dans les conduits.

### Exemple 1

L'exemple qui suit est destiné à illustrer l'invention.
Il concerne l'homopolymérisation en suspension aqueuse du chlorure de vinyle à l'aide de peroxydicarbonate de diéthyle en solution à 30 % en poids environ dans l'adipate de diéthylhexyle. Le peroxydicarbonate est fabriqué au départ de chloroformiate d'éthyle, de peroxyde d'hydrogène et d'hydroxyde de sodium, avant d'être extrait par de l'adipate de diéthylhexyle.

### Préparation de la solution de peroxydicarbonate de diéthyle.

Dans un réacteur agité de 1000 l refroidi sous 10 °C, on introduit 622 kg d'une solution aqueuse prérefroidie à 5 °C de chlorure de sodium à 180 g/kg (soit 510 kg d'eau déminéralisée et 112 kg de NaCl). On introduit ensuite successivement dans la solution aqueuse agitée 20,4 kg de chloroformiate d'éthyle et 8,5 kg de solution aqueuse de peroxyde d'hydrogène à 350 g/kg et enfin, très lentement, 36,1 1 de solution aqueuse d'hydroxyde de sodium à 200 g/kg de façon à maintenir la température sous 10°C. La densité du milieu aqueux de réaction s'élève à 1,11. 10 minutes après la fin de l'introduction de la solution de NaOH, on introduit 34,5 kg d'adipate de diéthylhexyle prérefroidi à 5 °C. Après avoir maintenu le milieu réactionnel sous agitation pendant 15 minutes en refroidissant à 5 °C, on stoppe l'agitation. On sépare ensuite par décantation la phase aqueuse (phase dense) et on récupére la phase organique. La solution de peroxydicarbonate de diéthyle dans l'adipate de diéthylhexyle ainsi produite est stockée à 5 °C en vue de son utilisation ultérieure. Sa teneur en peroxydicarbonate de diéthyle (évaluée par analyse) s'élève à 287 g/kg.

### Polymérisation du chlorure de vinyle.

Dans un réacteur d'une capacité de 3,9 m³, équipé d'un agitateur et d'une double enveloppe, on introduit à température ambiante et sous agitation (50t/min) 1869 kg d'eau déminéralisée, 0,801 kg d'alcool polyvinylique (taux d'hydrolyse 72 % molaires) et 0,534 kg d'alcool polyvinylique (taux d'hydrolyse 55 % molaires), 1,793 kg de la solution d'initiateur préparée telle que ci-dessus (soit 0,515 kg de peroxydicarbonate de diéthyle). On ferme le réacteur, on arrête l'agitation et on met le réacteur sous un vide partiel (60mm Hg absolus) que l'on maintient pendant 5 minutes. On remet l'agitation en marche (110t/min) et on introduit alors 1335 kg de chlorure de vinyle. On chauffe le milieu à 53 °C, après quoi on fait circuler de l'eau froide dans la double enveloppe. Le moment où le milieu de polymérisation atteint 53 °C est considéré comme le début de la polymérisation (temps=to). Après 6h de marche (comptés à partir de to), la pression dans le réacteur a baissé de 1,5kg/cm². On arrête la polymérisation en effectuant successivement: une introduction de 0,35 kg d'ammoniac, le dégazage du chlorure de vinyle non converti et le refroidissement. Le polychlorure de vinyle produit est isolé de manière conventionnelle de la suspension aqueuse. On recueille 1118 kg de PVC dont le nombre K (à 20 °C dans la cyclohexanone à 5 g/l) s'élève à 71.0.

Le tableau ci-dessous récapitule les propriétés évaluées sur le PVC produit: nombre K (à 20 °C dans la cyclohexanone à 5 g/l); masse volumique apparente (MVAE), porosité (% d'absorption de diéthylhexylphtalate), granulométrie et, enfin, nombre d'yeux-de-poisson (fish-eyes) exprimé en points par dm² et évalué sur un film extrudé au départ d'un mélange de 100 parties en poids de PVC et 40 parties de phtalate de diéthylhexyle.

### Exemple 2 (comparatif)

A titre comparatif, on a reproduit la polymérisation du chlorure de vinyle dans les mêmes conditions qu'à l'exemple 1, si ce n'est qu'on synthétise d'abord la quantité appropriée de peroxydicarbonate de diéthyle in situ dans le réacteur de polymérisation par mise en réaction à température ambiante et sous agitation de 0,734 kg de chloroformiate d'éthyle et de 0,109 kg de peroxyde d'hydrogène en présence de la quantité totale d'eau (alcalinisée par addition de 0,284 kg d'hydroxyde de sodium) et de la quantité totale d'alcools polyvinyliques destinés à la polymérisation (cf exemple 1: soit 1860 kg d'eau et au total 1,335 kg d'alcools polyvinyliques). En fin de synthèse "in situ" de l'initiateur, on ferme le réacteur, on arrête l'agitation, on met le réacteur sous vide partiel (60mm Hg absolus) pendant 5 minutes et on introduit sous agitation (110t/min) 1335 kg de chlorure de vinyle. Ensuite, on a procédé à la mise en chauffe et à la polymérisation comme à l'exemple 1. Après 5h51min, la pression dans le réacteur a baissé de 1,5 kg/cm² et on a arrêté la polymérisation. On recueille 1092 kg de PVC dont le nombre K (mesuré dans les mêmes conditions) s'élève à 71,3.

Le tableau ci-dessous récapitule également les propriétés évaluées sur le PVC produit selon l'exemple comparatif 2.

De la comparaison des résultats figurant au tableau, il apparaît que la mise en oeuvre du peroxydicarbonate de diéthyle en solution dans l'adipate de diéthylhexyle (selon l'invention) n'a pas d'effet significatif sur la cinétique de polymérisation, ni sur les propriétés générales du PVC produit. En outre, le film extrudé au départ du PVC produit selon l'invention (exemple 1) présente un nombre significativement réduit d'yeux-de-poisson par comparaison avec un film extrudé au départ de PVC produit à l'aide de peroxydicarbonate de diéthyle produit "in situ" (exemple 2, comparatif).

**Tableau**

| Numéro de l'exemple | 1 | 2 |
|---|---|---|
| Durée totale de polymérisation, h. min. | 6.00 | 5.51 |
| Nombre K | 71,0 | 71,3 |
| MVAE, kg/l | 0,484 | 0,486 |
| Porosité, % | 33,3 | 32,5 |

| Granulométrie, g/kg | | |
|---|---|---|
| > 250 µm | 4 | 5 |
| 177-250 µm | 57 | 72 |
| 125-177 µm | 461 | 507 |
| 88-125 µm | 422 | 369 |
| 63-88 µm | 54 | 46 |
| 45-63 µm | 2 | 1 |
| < 45 µm | 0 | 0 |
| Nombre d'yeux-de-poisson, pts/dm² | 8 | 44 |

## Revendications

1. Procédé de polymérisation en suspension aqueuse du chlorure de vinyle à l'aide de peroxydicarbonates de dialkyle dont les radicaux alkyles contiennent 2 ou 3 atomes de carbone, **caractérisé en ce que** le paroxydicarbonate de dialkyle est mis en oeuvre sous la forme d'une solution constituée essentiellement du peroxydicarbonate de dialkyle et d'un alkanedicarboxylate de dialkyle liquide et insoluble dans l'eau.

2. Procédé de polymérisation en suspension aqueuse du chlorure de vinyle selon la revendication 1, **caractérisé en ce que** la quantité de comonomères éventuellement mis en oeuvre avec le chlorure de vinyle n'excède pas 50 % en moles du mélange de tous les comonomères.

3. Procédé de polymérisation en suspension aqueuse du chlorure de vinyle selon la revendication 1, **caractérisé en ce que** l'alkanedicarboxylate de dialkyle est choisi parmi les esters liquides dérivés d'acides alkanedicarboxyliques en C₄ à C₁₀ et d'alkanols en C₂ à C₁₂.

4. Procédé de polymérisation en suspension aqueuse du chlorure de vinyle selon la revendication 3, **caractérisé en ce que** l'alkanedicarboxylate de dialkyle est choisi parmi les hexanedicarboxylates (adipates) dérivés d'acide adipique et d'alkanols en C₆ à C₁₀.

5. Procédé de polymérisation en suspension aqueuse du chlorure de vinyle selon la revendication 1, **caractérisé en ce que** la concentration en peroxydicarbonate de dialkyle de la solution s'élève à 15 à 40 % en poids.

6. Procédé de polymérisation en suspension aqueuse du chlorure de vinyle selon les revendications 1 à 5, **caractérisé en ce que** on met en oeuvre du peroxydicarbonate de diéthyle ou de diisopropyle sous la forme d'une solution dans un hexanedicarboxylate (adipate) dérivé d'acide adipique et d'un alkanol en C₆ à C₁₀.

7. Procédé de polymérisation en suspension aqueuse du chlorure de vinyle selon les revendications 1 à 6, **caractérisé en ce qu'**on initie la polymérisation exclusivement à l'aide de peroxydicarbonates de dialkyle dont les radicaux alkyles contiennent 2 ou 3 atomes de carbone.

8. Procédé de fabrication d'une solution de peroxydicarbonate de dialkyle dont les radicaux alkyles contiennent 2 ou 3 atomes de carbone, **caractérisé en ce que**, dans une première étape, on fabrique un peroxydicarbonate de dialkyle dont les radicaux alkyles contiennent 2 ou 3 atomes de carbone par mise en réaction dans l'eau de quantités appropriées d'halogénoformiate d'alkyle avec un peroxyde inorganique en présence d'un sel inorganique ajouté en quantité suffisante pour augmenter la densité du milieu aqueux de réaction et, dans une deuxième étape, on sépare le peroxydicarbonate de dialkyle fabriqué par extraction au moyen d'un solvant insoluble dans l'eau pour produire une solution de peroxydicarbonate de dialkyle dans ce solvant; le procédé étant **caractérisé en outre en ce qu'**on ajoute le solvant d'extraction au milieu de réaction aqueux après terminaison de la réaction de fabrication du peroxydicarbonate de dialkyle, on laisse décanter les phases et on sépare la phase organique surnageante de la phase aqueuse de réaction pour recueillir une solution pure de peroxydicarbonate.

9. Procédé de fabrication d'une solution de peroxydicarbonate de dialkyle dont les radicaux alkyles contiennent 2 ou 3 atomes de carbone selon la revendication 8, **caractérisé en ce qu'**on met en oeuvre le sel inorganique en une quantité suffisante pour amener la densité du milieu aqueux de réaction à une valeur au moins égale à 1,05.

10. Procédé de fabrication d'une solution de peroxydicarbonate de dialkyle dont les radicaux alkyles contiennent 2 ou 3 atomes de carbone selon les revendications 8 et 9, **caractérisé en ce que** le sel inorganique est du chlorure de sodium.

11. Procédé de fabrication d'une solution de peroxydicarbonate de dialkyle dont les radicaux alkyles contiennent 2 ou 3 atomes de carbone selon la revendication 8, **caractérisé en ce que** le solvant insoluble dans l'eau est choisi parmi les composés organiques insolubles dans l'eau choisis parmi les plastifiants usuels du polychlorure de vinyle.

12. Procédé de fabrication d'une solution de peroxydicarbonate de dialkyle dont les radicaux alkyles contiennent 2 ou 3 atomes de carbone selon la revendication 11, **caractérisé en ce que** le solvant insoluble dans l'eau est choisi parmi les alkanedicarboxylates de dialkyle dérivés d'acides alkanedicarboxyliques en C₄ à C₈ et d'alkanols en C₆ à C₁₀.

13. Procédé de fabrication d'une solution de peroxydicarbonate de dialkyle dont les radicaux alkyles contiennent 2 ou 3 atomes de carbone selon la revendication 12, **caractérisé en ce que** le solvant insoluble dans l'eau est choisi parmi les hexanedicarboxylates (adipates) dérivés d'acide adipique et d'un alkanol en C₆ à C₁₀.

14. Procédé de fabrication d'une solution de peroxydicarbonate de dialkyle dont les radicaux alkyles contiennent 2 ou 3 atomes de carbone, **caractérisé en ce que**, dans une première étape, on fabrique un peroxydicarbonate de dialkyle dont les radicaux alkyles contiennent 2 ou 3 atomes de carbone par mise en réaction dans l'eau de quantités appropriées d'halogénioformiate d'alkyle avec un peroxyde inorganique en présence d'un sel inorganique en quantité suffisante pour augmenter la densité du milieu aqueux de réaction et, dans une deuxième étape, on sépare le peroxydicarbonate de dialkyle fabriqué par extraction au moyen d'un solvant insoluble dans l'eau, choisi parmi les composés organiques insolubles dans l'eau choisis parmi les plastifiants usuels du polychlorure de vinyle, pour produire une solution de peroxydicarbonate de dialkyle dans ce solvant.

## Claims

1. Process for the aqueous suspension polymerization of vinyl chloride with the use of dialkyl peroxydicarbonates in which the alkyl radicals contain 2 or 3 carbon atoms, **characterized in that** the dialkyl peroxydicarbonate is used in the form of a solution consisting essentially of the dialkyl peroxydicarbonate and of a dialkyl alkanedicarboxylate which is liquid and insoluble in water.

2. Process for the aqueous suspension polymerization of vinyl chloride according to Claim 1, **characterized in that** the quantity of comonomers that are optionally used with the vinyl chloride does not exceed 50 mol% of the mixture of all the comonomers.

3. Process for the aqueous suspension polymerization of vinyl chloride according to Claim 1, **characterized in that** the dialkyl alkanedicarboxylate is chosen from the liquid esters derived from C₄-C₁₀ alkanedicarboxylic acids and from C₂-C₁₂ alkanols.

4. Process for the aqueous suspension polymerization of vinyl chloride according to Claim 3, **characterized in that** the dialkyl alkanedicarboxylate is chosen from hexanedicarboxylates (adipates) derived from adipic acid and from C₆-C₁₀ alkanols.

5. Process for the aqueous suspension polymerization of vinyl chloride according to Claim 1, **characterized in that** the concentration of dialkyl peroxydicarbonate in the solution is from 15 to 40 % by weight.

6. Process for the aqueous suspension polymerization of vinyl chloride according to Claims 1 to 5, **characterized in that** diethyl or diisopropyl peroxydicarbonate is used in the form of a solution in a hexanedicarboxylate (adipate) derived from adipic acid and from a C₆-C₁₀ alkanol.

7. Process for the aqueous suspension polymerization of vinyl chloride according to Claims 1 to 6, **characterized in that** the polymerization is initiated exclusively with the use of dialkyl peroxydicarbonates in which the alkyl radicals contain 2 or 3 carbon atoms.

8. Process for the manufacture of a solution of dialkyl peroxydicarbonate in which the alkyl radicals contain 2 or 3 carbon atoms, **characterized in that**, in a first stage, a dialkyl peroxydicarbonate in which the alkyl radicals contain 2 or 3 carbon atoms is manufactured by reacting, in water, appropriate quantities of alkyl haloformate with an inorganic peroxide in the presence of an inorganic salt added in sufficient quantity to increase the density of the aqueous reaction mixture and, in a second stage, the dialkyl peroxydicarbonate manufactured is isolated by extraction by means of a water-insoluble solvent in order to produce a solution of dialkyl peroxydicarbonate in this solvent; the process being further **characterized in that** the extraction solvent is added to the aqueous reaction mixture after the reaction of manufacture of the dialkyl peroxydicarbonate is finished, the phases are allowed to settle out and the supernatant organic phase is separated from the aqueous reaction phase in order to collect a pure peroxydicarbonate solution.

9. Process for the manufacture of a solution of dialkyl peroxydicarbonate in which the alkyl radicals contain 2 or 3 carbon atoms according to Claim 8, **characterized in that** the inorganic salt is used in a quantity which is sufficient to bring the relative density of the aqueous reaction medium to a value of at least 1.05.

10. Process for the manufacture of a solution of dialkyl peroxydicarbonate in which the alkyl radicals contain 2 or 3 carbon atoms according to Claims 8 and 9, **characterized in that** the inorganic salt is sodium chloride.

11. Process for the manufacture of a solution of dialkyl peroxydicarbonate in which the alkyl radicals contain 2 or 3 carbon atoms according to Claim 8, **characterized in that** the water-insoluble solvent is chosen from water-insoluble organic compounds chosen from the usual plasticizers for polyvinyl chloride.

12. Process for the manufacture of a solution of dialkyl peroxydicarbonate in which the alkyl radicals contain 2 or 3 carbon atoms according to Claim 11, **characterized in that** the water-insoluble solvent is chosen from dialkyl alkanedicarboxylates derived from C₄-C₈ alkanedicarboxylic acids and from C₆-C₁₀ alkanols.

13. Process for the manufacture of a solution of dialkyl peroxydicarbonate in which the alkyl radicals contain 2 or 3 carbon atoms according to Claim 12, **characterized in that** the water-insoluble solvent is chosen from hexanedicarboxylates (adipates) derived from adipic acid and from a C₆-C₁₀ alkanol.

14. Process for the manufacture of a solution of dialkyl peroxydicarbonate in which the alkyl radicals contain 2 or 3 carbon atoms, **characterized in that**, in a first stage, a dialkyl peroxydicarbonate in which the alkyl radicals contain 2 or 3 carbon atoms is manufactured by reacting, in water, appropriate quantities of alkyl haloformate with an inorganic peroxide in the presence of an inorganic salt in sufficient quantity to increase the density of the aqueous reaction mixture and, in a second stage, the dialkyl peroxydicarbonate manufactured is isolated by extraction by means of a water-insoluble solvent chosen from water-insoluble organic compounds chosen from the usual plasticizers for polyvinyl chloride, in order to produce a solution of dialkyl peroxydicarbonate in this solvent.

## Patentansprüche

1. Verfahren zur wäßrigen Suspensionspolymerisation von Vinylchlorid mittels Dialkylperoxydicarbonaten, deren Alkylreste 2 oder 3 Kohlenstoffatome enthalten, **dadurch gekennzeichnet, daß** das Dialkylperoxydicarbonat in Form einer Lösung verwendet wird, die im wesentlichen aus dem Dialkylperoxydicarbonat und einem flüssigen und in Wasser unlöslichen Dialkylalkandicarboxylat besteht.

2. Verfahren zur wäßrigen Suspensionspolymerisation von Vinylchlorid gemäß Anspruch 1, **dadurch gekennzeichnet, daß** die Menge an gegebenenfalls mit dem Vinylchlorid verwendeten Comonomeren 50 Mol-% des Gemischs aller Comonomere nicht übersteigt.

3. Verfahren zur wäßrigen Suspensionspolymerisation von Vinylchlorid gemäß Anspruch 1, **dadurch gekennzeichnet, daß** das Dialkylalkandicarboxylat unter den flüssigen Estern, die sich von C4- bis C₁₀-Alkandicarbonsäuren und C2- bis C12-Alkanolen ableiten, ausgewählt wird.

4. Verfahren zur wäßrigen Suspensionspolymerisation von Vinylchlorid gemäß Anspruch 3, **dadurch gekennzeichnet, daß** das Dialkylalkandicarboxylat unter den Hexandicarboxylaten (Adipaten), die sich von Adipinsäure und C6- bis C10-Alkanolen ableiten, ausgewählt wird.

5. Verfahren zur wäßrigen Suspensionspolymerisation von Vinylchlorid gemäß Anspruch 1, **dadurch gekennzeichnet, daß** die Konzentration der Lösung an Dialkylperoxydicarbonat 15 bis 40 Gew.-% beträgt.

6. Verfahren zur wäßrigen Suspensionspolymerisation von Vinylchlorid gemäß den Ansprüchen 1 bis 5, **dadurch gekennzeichnet, daß** man Diethyl- oder Diisopropylperoxydicarbonat in Form einer Lösung in einem Hexandicarboxylat (Adipat), das sich von Adipinsäure und einem C6- bis C10-Alkanol ableitet, verwendet.

7. Verfahren zur wäßrigen Suspensionspolymerisation von Vinylchlorid gemäß den Ansprüchen 1 bis 6, **dadurch gekennzeichnet, daß** man die Polymerisation ausschließlich mittels Dialkylperoxydicarbonaten, deren Alkylreste 2 oder 3 Kohlenstoffatome enthalten, initiiert.

8. Verfahren zur Herstellung einer Lösung von Dialkylperoxydicarbonat, dessen Alkylreste 2 oder 3 Kohlenstoffatome enthalten, **dadurch gekennzeichnet, daß** man in einem ersten Schritt ein Dialkylperoxydicarbonat, dessen Alkylreste 2 oder 3 Kohlenstoffatome enthalten, durch Umsetzen von geeigneten Mengen an Alkylhalogenformiat mit einem anorganischen Peroxid in Wasser in Gegenwart eines anorganischen Salzes, das in einer Menge zugefügt wird, die ausreicht, um die Dichte des wäßrigen Reaktionsmediums zu erhöhen, herstellt und man in einem zweiten Schritt das hergestellte Dialkylperoxydicarbonat durch Extraktion mittels eines in Wasser unlöslichen Lösungsmittels abtrennt, wobei man eine Lösung von Dialkylperoxydicarbonat in diesem Lösungsmittel erhält, wobei das Verfahren außerdem **dadurch gekennzeichnet ist, daß** man das Extraktionslösungsmittel nach Beendigung der Umsetzung zur Herstellung des Dialkylperoxydicarbonats zu dem wäßrigen Reaktionsmedium gibt, die Phasen trennen läßt und die obere organische Phase von der wäßrigen Reaktionsphase abtrennt, wobei man eine reine Lösung von Peroxydicarbonat erhält.

9. Verfahren zur Herstellung einer Lösung von Dialkylperoxydicarbonat, dessen Alkylreste 2 oder 3 Kohlenstoffatome enthalten, gemäß Anspruch 8, **dadurch gekennzeichnet, daß** man das anorganische Salz in einer Menge einsetzt, die ausreicht, um die Dichte des wäßrigen Reaktionsmediums auf einen Wert von wenigstens gleich 1,05 zu bringen.

10. Verfahren zur Herstellung einer Lösung von Dialkylperoxydicarbonat, dessen Alkylreste 2 oder 3 Kohlenstoffatome enthalten, gemäß den Ansprüchen 8 und 9, **dadurch gekennzeichnet, daß** das anorganische Salz Natriumchlorid ist.

11. Verfahren zur Herstellung einer Lösung von Dialkylperoxydicarbonat, dessen Alkylreste 2 oder 3 Kohlenstoffatome enthalten, gemäß Anspruch 8, **dadurch gekennzeichnet, daß** das in Wasser unlösliche Lösungsmittel unter den in Wasser unlöslichen organischen Verbindungen, die unter den üblichen Weichmachern für Polyvinylchlorid ausgewählt werden, ausgewählt wird.

12. Verfahren zur Herstellung einer Lösung von Dialkylperoxydicarbonat, dessen Alkylreste 2 oder 3 Kohlenstoffatome enthalten, gemäß Anspruch 11, **dadurch gekennzeichnet, daß** das in Wasser unlösliche Lösungsmittel unter den Dialkylalkandicarboxylaten, die sich von C4-bis C8-Alkandicarbonsäuren und C6- bis C10-Alkanolen ableiten, ausgewählt wird.

13. Verfahren zur Herstellung einer Lösung von Dialkylperoxydicarbonat, dessen Alkylreste 2 oder 3 Kohlenstoffatome enthalten, gemäß Anspruch 12, **dadurch gekennzeichnet, daß** das in Wasser unlösliche Lösungsmittel unter den Hexandicarboxylaten (Adipaten), die sich von Adipinsäure und einem C6- bis C10-Alkanol ableiten, ausgewählt wird.

14. Verfahren zur Herstellung einer Lösung von Dialkylperoxydicarbonat, dessen Alkylreste 2 oder 3 Kohlenstoffatome enthalten, **dadurch gekennzeichnet, daß** man in einem ersten Schritt ein Dialkylperoxydicarbonat, dessen Alkylreste 2 oder 3 Kohlenstoffatome enthalten, durch Umsetzen von geeigneten Mengen an Alkylhalogenformiat mit einem anorganischen Peroxid in Wasser in Gegenwart eines anorganischen Salzes in ausreichender Menge, um die Dichte des wäßrigen Reaktionsmediums zu erhöhen, herstellt und man in einem zweiten Schritt das hergestellte Dialkylperoxydicarbonat durch Extraktion mittels eines in Wasser unlöslichen Lösungsmittels, das unter den in Wasser unlöslichen organischen Verbindungen, die unter den üblichen Weichmachern für Polyvinylchlorid ausgewählt werden, ausgewählt wird, abtrennt, wobei man eine Lösung von Dialkylperoxydicarbonat in diesem Lösungsmittel erhält.
